# EUROPEAN PATENT SPECIFICATION

(11) **EP 2 255 002 B1**
(45) Date of publication and mention of the grant of the patent: **02.04.2014**
(21) Application number: 09709670.5
(22) Date of filing: 09.02.2009
(51) Int. Cl.: C12N 15/11

(54) **METHOD TO INHIBIT RIBONUCLEASE DICER, RIBONUCLEASE DICER INHIBITOR, AND USE OF RNA APTAMERS AS RIBONUCLEASE DICER INHIBITORS**
VERFAHREN ZUR HEMMUNG VON RIBONUKLEASE DICER, RIBONUKLEASE-DICER-INHIBITOR UND VERWENDUNG VON RNA-APTAMEREN ALS RIBONUKLEASE-DICER-INHIBITOREN
PROCÉDÉ POUR INHIBER LA RIBONUCLÉASE DICER, INHIBITEUR DE LA RIBONUCLÉASE DICER ET UTILISATION D APTAMÈRES D ARN EN TANT QU INHIBITEURS DE LA RIBONUCLÉASE DICER

(30) Priority: 14.02.2008 PL 38445508
(43) Date of publication of application: 01.12.2010
(73) Proprietor: Instytut Chemii Bioorganicznej PAN, 61-704 Poznan (PL)
(72) Inventor: FIGLEROWICZ, Marek, 62-023 Gadki (PL); TYCZEWSKA, Agata, 62-023 Gadki (PL); TWARDOWSKI, Tomasz, 62-090 Kiekrz (PL); SZOPA, Aleksandra, 97-220 Rzeczyca (PL); KIETRYS, Anna, 62-064 Plewiska (PL)
(74) Representative: Rzazewska, Dorota
(86) International application number: PCT/PL2009/000013
(87) International publication number: WO 2009/102225

(56) References cited:
- WO-A-2004/035796
- WO-A-2007/043784
- US-A1- 2006 064 769
- ULRICH HENNING: "DNA AND RNA APTAMERS AS MODULATORS OF PROTEIN FUNCTION" MEDICINAL CHEMISTRY, BENTHAM SCIENCE PUBLISHERS LTD, BUSSUM, NL, vol. 1, no. 2, 1 March 2005 (2005-03-01), pages 199-208, XP001248936 ISSN: 1573-4064
- JASKIEWICZ LUKASZ ET AL: "Role of Dicer in posttranscriptional RNA silencing" CURRENT TOPICS IN MICROBIOLOGY AND IMMUNOLOGY, SPRINGER, BERLIN, DE, vol. 320, 1 January 2008 (2008-01-01), pages 77-97, XP009121485 ISSN: 0070-217X

## Description

The subject of the present invention is an in vitro method to inhibit ribonuclease Dicer, ribonuclease Dicer inhibitor, and use of RNA aptamers to inhibit ribonuclease Dicer. More specifically, this solution relates to using RNA aptamers as competitive ribonuclease Dicer inhibitors, use of RNA aptamers as allosteric ribonuclease Dicer inhibitors, and use of RNA aptamers as selective inhibitors of emergence of the selected miRNAs.

Aptamers are relatively short RNA or DNA molecules, which bind with a strictly defined molecule with high affinity and specificity. The word "aptamer" comes from Latin "aptus", i.e. "match" [Tuerk, C. and L. Gold, Systematic evolution of ligands by exponential enrichment: RNA ligands to bacteriophage T4 DNA polymerase. Science, 1990. 249(4968): p. 505-10; Ellington, A.D. and J.W. Szostak, In vitro selection of RNA molecules that bind specific ligands. Nature, 1990. 346(6287): p. 818-22]. One can say that the aptamer is a ligand capable of binding some specific molecule e.g. a protein, sugar, amino acid, nucleotide, antibiotic, or vitamin. Up till now, a substantial number of various aptamers has been obtained. They were selected as a ligands binding a wide spechum of individuals, from small inorganic molecules to single-cell organisms [Blank, M. and M. Blind, Aptamers as tools for target validation. Curr Opin Chem Biol, 2005. 9(4): p. 336-42; Nimjee, S.M., C.P. Rusconi, and B.A. Sullenger, Aptamers: an emerging class of therapeutics. Annu Rev Med, 2005. 56: p. 555-83; Yan, A.C., et al., Aptamers: prospects in therapeutics and biomedicine. Front Biosci, 2005. 10: p. 1802-27; Proske, D., et al., Aptamers--basic research, drug development, and clinical applications. Appl Microbiol Biotechnol, 2005. 69(4): p. 367-74]. Specificity and high binding force (dissociation constant amounting to nano- or picomoles) results in aptamers being often compared to antibodies. Aptamer molecular mass is 10-15 kDa on average, i.e. about 10 times less than the mass of a single antibody [White, R.R., B.A. Sullenger, and C.P. Rusconi, Developing aptamers into therapeutics. J Clin Invest, 2000. 106(8): p. 929-34], which makes it easier for them to be spread within the organism and to be promptly removed from the blood circulation system.

Aptamers are usually obtained by *in vitro* selection that is called the SELEX (Systematic Evolution of Ligands by Exponential Enrichment) method. The method makes it possible to identify the molecules that possess a desired property, e.g. strong binding with a selected molecule (target molecule - TM), from the input pool of many RNA or DNA molecules (molecules with an undefined sequence, i.e. random sequence). The input pool of RNA or DNA molecules is called a combinatorial library. It is a set of all oligonucleotides with definite length n that are possible to be synthesised. In practice, the oligonucleotides being selected, beside the centrally situated random sequence, also contain the so-called flanking regions with known sequences. They are used to amplify and clone the identified molecules. The number of oligonucleotides that create the full combinatorial library (complete input pool) depends on random sequence's length. In each of its positions, one of the four nucleotides: A, G, C, or T/U can occur. Therefore, in case of n-nt random sequence, it is possible to synthesise 4ⁿ of various oligonucleotides.

Typical RNA aptamer selection takes place according to the following scheme. Initially, a possibly high number of single-strand DNA molecules that contain n-nt random sequence is obtained by chemical synthesis. These molecules are then converted to the double-strand DNA, which serves as a template to synthesise a combinatorial library of single-strand RNA molecules, by the *in vitro* transcription method. The RNA obtained is incubated with a selected target molecule (TM). Then, all non-bound RNA molecules are removed. At the next stage, molecules bound with TM are isolated. The RNAs thus obtained are converted to the double-strand DNA by the RT-PCR method involving starters that are complementary to flanking sequences. The product obtained serves as a template to synthesise the narrowed RNA pool by *in vitro* transcription. The molecules obtained then undergo the selection process again. Upon having conducted the planned number of selection cycles, dsDNA is ligated into the plasmid and cloned in bacterial cells. Finally, individual clones are sequenced. In this way, the sequences of RNA molecules that bind with TM are identified.

To read out the information that is stored in genomes of living organisms is one of the most important tasks faced by contemporary molecular biology and bioinformatics. It was proved that a single gene could be a source of more than one type of transcript. What is more, many different mRNAs may arise from a single type of transcript. No observation was made that there existed any simple correlation between genome size and complexity of the organism. Only in bacteria and simple animal or plant organisms, most of the genome encodes proteins. In higher organisms, protein-encoding sequences constitute only a small part of the genetic material (less than 5% in humans). Due to the fact that the human genome's full sequence had been identified, it was acknowledged that, contrary to what had been expected earlier, it did not contain 150 thousand, but only about 25 thousand genes, i.e. more or less the same amount as can be found in the simple model plant, *Arabidopsis thaliana.* Nevertheless, a real turning point in research concerning mechanisms that govern the expression of genetic information has taken place only recently, chiefly due to an unexpected discovery of the phenomenon called RNA interference (RNAi) and of short regulatory RNAs, including micro RNAs (miRNAs) [Fire, A., et al., Potent and specific genetic interference by double-stranded RNA in Caenorhabditis elegans. Nature, 1998. 391(6669): p. 806-11]. It was found that sequences that encode regulatory RNA molecules, the so-called miRNAs, are present in genomes of eukaryotic organisms, and thus in the human genome too. Such sequences may both occur within protein coding genes (often in introns), and create independent genes that do not encode proteins but only RNA. miRNA containing transcripts are called pri-miRNA. In their structures, there usually occur about 50-80 nucleotidie long, not fully complementary double-strand regions, which, due to their shape, are referred to as the hairpin type structures. It was observed that miRNAs arise both in plants and animals, but their maturing process is different in both types of organisms. In case of human cells, three basic stages of miRNA biogenesis can be distinguished [Bartel, D.P., MicroRNAs: genomics, biogenesis, mechanism, and function. Cell, 2004. 116(2): p. 281-97]. At the first one, the enzyme called Drosha cuts a double-strand fragment (hairpin structure) out of the pri-miRNA. At the second stage, the newly emerged 50-80 nucleotide (nt) molecule - pre-miRNA is transported from nucleus to cytoplasm by exportin 5. At the third stage, pre-miRNA is recognised by ribonuclease Dicer, which cuts a 20-23 nt duplex out of it. Then, the duplex is conveyed into another protein complex RISC (RNA-induced silencing complex). At the next stage, RISC is activated. This process consists in removing one strand of the duplex, whereas the other one, called miRNA, remains within the complex, and serves as a probe that makes it possible to specifically recognize fully or partially complementary RNA molecules [Nykanen, A., B. Haley, and P.D. Zamore, ATP requirements and small interfering RNA structure in the RNA interference pathway. Cell, 2001. 107(3): p. 309-21; Hammond; S.M., et al., An RNA-directed nuclease mediates post-transcriptional gene silencing in Drosophila cells. Nature, 2000. 404(6775): p. 293-6]. The active RISC complex can bind with mRNA. If mRNA is fully complementary to the miRNA that is present in the RISC complex, then it is cut (more or less in the middle of the mRNA/siRNA duplex) [Haley, B. and P.D. Zamore, Kinetic analysis of the RNAi enzyme complex. Nat Struct Mol Biol, 2004. 11(7): p. 599-606]. As a consequence, within a relatively short time, the whole mRNA pool becomes degraded, and the so-called posttranscriptional gene silencing (PTGS), takes place. However, if mRNA is only partially complementary to the miRNA that is present in the RISC complex, then it is not degraded, but remains bound with the protein complex, and thus it cannot serve as a template for protein synthesis. As a result, the gene is silenced at the level of translation (TR - translational repression).

It was proved that miRNAs perform some very important roles both in many physiological processes (e.g. developmental processes: cell differentiation, apoptosis, maintaining the line of maternal cells or brain morphogenesis) and pathological processes (cancerous transformation, neurodegenerative processes, etc.) [Mattick, J.S. and I.V. Makunin, Small regulatory RNAs in mammals. Hum Mol Genet, 2005. 14 Spec No 1: p. R121-32; Croce, C.M. and G.A. Calin, miRNAs, cancer, and stem cell division. Cell, 2005. 122(1): p. 6-7; Giraldez, A.J., et al., MicroRNAs regulate brain morphogenesis in zebrafish. Science, 2005. 308(5723): p. 833-8]. Results of some of the most resent research indicate that about 30 - 40% of all human genes are controlled by miRNA.

Ribonuclease Dicer is a member of the RNase III family, i.e. those endoribonucleases that specifically cut double-strand RNA molecules. All enzymes that belong to this family are characterised by the presence of one or two ribonuclease domains, called the RNase III domain. As a result of dsRNA digesing by RNases III, short duplexes are generated. They possess the phosphate group at the 5' ends, and two non-paired nucleotides at the 3' ends. RNases III differ in size, and they may have 200 to 2000 amino acids. The data that has been collected so far suggests that only one Dicer nuclease is encoded in the human genome. In case of miRNA biogenesis, ribonuclease Dicer is responsible for cutting 20-23 nt miRNA - containing duplexes, out of precursor pre-miRNAs. Based on biochemical data, it has been found out that human ribonuclease Dicer and bacterial RNase III contains only one active centre, in which both of the RNA strands are cut [Zhang, K. and A.W. Nicholson, Regulation of ribonuclease III processing by double-helical sequence antideterminants. Proc Natl Acad Sci USA, 1997. 94(25): p. 13437-41; Macrae, I.J., et al:, Structural basis for double-stranded RNA processing by Dicer. Science, 2006. 311(5758): p. 195-8]. It was also proved, however, that in case of human Dicer, two RNase III domains, which make it up, act independently from each other (i.e. each of them cuts only one of the strands) [Macrae, I.J., et al.].

There are many examples that indicate that RNA aptamers may be successfully used as regulators of processes that take place in living organisms. Some of them have already been introduced as medicines into the market, for instance in the therapy of the disease called AMD (Age Macular Degeneration). The main factor that causes blood vessel hypertrophy and effusions in the AMD disease is one of the isoforms of the vascular endothelial growth factor - VEGF₁₆₅ [Ferrara, N., H.P. Gerber, and J. LeCouter, The biology of VEGF and its receptors. Nat Med, 2003. 9(6): p. 669-76] . RNA aptamer selection was conducted on VEGF₁₆₅, and 3 molecules were obtained, which bound themselves specifically with VEGF₁₆₅ (K_{d+}49-130 pM) [Ruckman, J., et al., 2'-Fluoropyrimidine RNA-based aptamers to the 165-amino acid form of vascular endothelial growth factor (VEGF165). Inhibition of receptor binding and VEGF-induced vascular permeability through interactions requiring the exon 7-encoded domain. J Biol Chem, 1998. 273(32): p. 20556-67]. One of these aptamers, 27 nt NX-1838, is a strong angiogenesis inhibitor, and it has been used to produce the medicine called Macugen® [Siddiqui, M.A. and G.M. Keating, Pegaptanib: in exudative age-related macular degeneration. Drugs, 2005. 65(11): p. 1571-7; discussion 1578-9; Cunningham, E.T., Jr., et al., A phase II randomized double-masked trial of pegaptanib, an anti-vascular endothelial growth factor aptamer, for diabetic macular edema. Ophthalmology, 2005. 112(10): p. 1747-57; Ng, E.W. and A.P. Adamis, Targeting angiogenesis, the underlying disorder in neovascular age-related macular degeneration. Can J Ophthalmol, 2005. 40(3): p. 352-68; Bell, C., et al., Oligonucleotide NX1838 inhibits VEGF165-mediated cellular responses in vitro. In Vitro Cell Dev Biol Anim, 1999. 35(9): p. 533-42]. It prevents VEGF from being bound by VEGFR1 and VEGFR2 receptors. Based upon clinical research, improvement of vision was noticed with 80% of patients within 3 months after the aptamer had been administered. In 2004, Macugen® was approved by FDA as a medicine for patients with Age Macular Degeneration [http://www.centerwatch.com/patient/drugs/areal3.html].

Also, impact of the NX-1838 upon Wilms tumour development was tested in the organism of a mouse. An 84% reduction in cancerous mass was noted as compared to the control. Furthermore, lower occurrence of lung metastasis (20%) was observed as compared to the control (60%) [Huang, J., et al., Highly specific antiangiogenic therapy is effective in suppressing growth of experimental Wilms tumors. J Pediatr Surg, 2001. 36(2): p. 357-61] , coupled with 53% inhibition of neuroblastoma growth [Kim, E.S., et al., Potent VEGF blockade causes regression of coopted vessels in a model of neuroblastoma. Proc Natl Acad Sci U S A, 2002. 99(17): p. 11399-404].

Also, some attempts were described to use aptamers in thrombosis treatments. Thrombosis is a disease that is connected with distortions in the blood-clotting process. It can lead to some serious damages of the heart and other organs. One of the factors that induce the blood-clotting process is thrombin. Therefore, DNA aptamers were obtained with a high degree of affinity towards thrombin (K_{d} between 2 and 200 nM). One of them extended blood-clotting time from 25 to 169 seconds. Furthermore, it was observed that this aptamer inhibited both the free thrombin and the one that caused blood to clot. It stops to be active shortly after having been administered into the organism, which prevents from the necessity to use antidote [Bock, L.C., et al., Selection of single-stranded DNA molecules that bind and inhibit human thrombin. Nature, 1992. 355(6360): p. 564-6].

Also, an RNA aptamer (called 9.3t) was obtained, which specifically binds factor IX (K_{d} below 3nM) that is a serine protease which takes part in thrombin creation from prothrombin. It was proved that the 9.3t significantly extended the blood-clotting time both *in vitro* and *in vivo.* Based on the 9.3t sequence, an antisense oligonucleotide (5-2C) was designed in such a way as to bind with the 9.3t and to block its activity. It was proved that when the 5-2C was added, the aptamer's anticoagulation activity would get promptly inhibited [Rusconi, C.P., et al., RNA aptamers as reversible antagonists of coagulation factor IXa. Nature, 2002. 419(6902): p. 90-4; Rusconi, C.P., et al., Antidote-mediated control of an anticoagulant aptamer in vivo. Nat Biotechnol, 2004. 22(11): p. 1423-8]. Tests were made to find out if the pair 9.3t aptamer - 5-2C antidote could be used during heart surgeries (with cardiopulmonary bypass) as a substitute for heparin and protoamine. To this aim, heparin (300 IU/kg) and 9.3tC aptamer (0.5 mg/kg) were administered to pigs, and upon restoring systemic blood circulation (after 60 minutes), respectively, protamine (Img/100 IU of heparin) or 5-2C (5 mg/kg) was administered in order to inhibit anticoagulation action of heparin or aptamer. It was proved that the aptamer could effectively substitute heparin, no blood clot emergence was observed; the 5-2C antidote administered was well tolerated by the organism, and, furthermore, it reversed the aptamer-caused anticoagulation effect promptly and effectively [Nimjee, S.M., et al., A novel antidote-controlled anticoagulant reduces thrombin generation and inflammation and improves cardiac function in cardiopulmonary bypass surgery. Mol Ther, 2006.14(3): p. 408-15].

Aptamers that bind tenascine-C (TN-C) were also identified. TN-C is a protein that occurs in the extracellular matrix. The gene that encodes it is expressed during foetal development, wound healing, regeneration of organs, and in pathological conditions [Erickson, H.P. and M.A. Bourdon, Tenascin: an extracellular matrix protein prominent in specialized embryonic tissues and tumors. Annu Rev Cell Biol, 1989. 5: p. 71-92; Dretkiewicz K, W.E., Rolle K, Nowak S, Żukiel L, Barciszewski J, Rola tenascyny-C w patogenezie. Neuroskop, 2005. 7: p. 19-26]. A high level of TN-C gene expression was observed in malicious cancers. *In vitro* selection was conducted on whole U251 glioblastomy cells with TN-C expression, and on purified TN-C protein. As a result, aptamer of the length of 39 nt, called TTA1, was obtained, with dissociation constant of 5nM [Hicke, BJ., et al., Tenascin-C aptamers are generated using tumor cells and purified protein. J Biol Chem, 2001. 276(52): p. 48644-54]. It was found out that TTA1 binds to TN-C fibrinogen-like domain. Possibilities were also tested to deliver radioactive isotopes and cytotoxic medicines to cancerous cells using the TTA1. To this aim, several different cancers were examined, i.e.: breast cancer, lung cancer, colon cancer, and glioblastomy cancer (while the TTA1 labelled was administered intravenously). It was found out that fast intake and removal of TTA1 from blood and tissues made it possible to clearly visualise cancers [Hicke, B.J., et al., Tumor targeting by an aptamer. J Nucl Med, 2006. 47(4): p. 668-78].

In prostate cancers, increased accumulation of Prostate-Specific Membrane Antigen (PSMA) was observed. It was proved that its concentration increased together with progress of the disease (prostate cancer), whereas the highest PSMA level occurred in case of cancers that are resistant to hormonal treatment and in case of metastatic cancers. PSMA emergence was also observed outside prostate, during blood vessel creation in various solid tumours [Bostwick, D.G., et al., Prostate specific membrane antigen expression in prostatic intraepithelial neoplasia and adenocarcinoma: a study of 184 cases. Cancer, 1998. 82(11): p. 2256-61; Gregorakis, A.K., E.H. Holmes, and G.P. Murphy, Prostate-specific membrane antigen: current and future utility. Semin Urol Oncol, 1998. 16(1): p. 2-12; Chang, S.S., et al., Five different anti-prostate-specific membrane antigen (PSMA) antibodies confirm PSMA expression in tumor-associated neovasculature. Cancer Res, 1999. 59(13): p. 3192-8; Silver, D.A., et al., Prostate-specific membrane antigen expression in normal and malignant human tissues. Clin Cancer Res, 1997. 3(1): p. 81-5; Nielsen GK, S.K., Trumbull K, Spaulding B, Welcher R., Immunohistochemical characterization of prostate specific membrane antigen expression in the vasculature of normal and neoplastic tissues. Modern Path, 2004.17: p. 326A]. *In vitro* selection was conducted on the extracellular PSMA-xPSM component. After six selection rounds, two aptamers were obtained that did not manifest any common sequential motifs, called xPSM-A9 and xPSM-A10 o K_{d}=11.9 nM [Lupold, S.E., et al., Identification and characterization of nuclease-stabilized RNA molecules that bind human prostate cancer cells via the prostate-specific membrane antigen. Cancer Res, 2002. 62(14): p. 4029-33].

Also, prion-binding aptamer (PrP) selection was carried out. The PrP is a protein, which may occur in two alternative spatial forms: normal PrP^{C} (which contains about 40% α-helix and just a few *β*-folds) and pathogenic PrP^{Sc} (which is built of approx. 30% α-helix and 45% *β*-folds) [Pan, K.M., et al., Conversion of alpha-helices into beta-sheets features in the formation of the scrapie prion proteins. Proc Natl Acad Sci USA, 1993. 90(23): p. 10962-6]. PrP^{Sc} proteins are able to aggregate and deposit in the brain, which, in effect, leads to functioning distortions in cells and their death [Prusiner, S.B., et al., Prion protein biology. Cell, 1998. 93(3): p. 337-48; Prusiner, S.B., Prions. Proc Natl Acad Sci U S A, 1998. 95(23): p. 13363-83]. As a result of aptamer selection, RNA molecules were obtained that specifically recognised PrP^{Sc} and PrP^{C} prion proteins in mouse, hamster, and cattle brain homogenates [Rhie, A., et al., Characterization of 2'-fluoro-RNA aptamers that bind preferentially to disease-associated conformations of prion protein and inhibit conversion. J Biol Chem, 2003.278(41): p. 39697-705].

As early as at the beginning of the 90s of the 20th century, the SELEX method was used to obtain aptamers binding to reverse transcriptase of the HIV-1 virus. It was found out that one of them (called 1.1) significantly lowered the HIV-1 RT polymerase activity, but it did not affect remaining RT activities nor reverse transcriptase of other retroviruses [Tuerk, C., S. MacDougal, and L. Gold, RNA pseudoknots that inhibit human immunodeficiency virus type 1 reverse transcriptase. Proc Natl Acad Sci USA, 1992. 89(15): p. 6988-92]. Aptamers were also selected on nsP10 protein of the SARS (Severe Acute Respiratory Syndrome) virus. The molecules obtained efficiently inhibited nsP10 helicase activity [Jang, K.J., Lee, N.R., Yeo, W.S., Jeong, Y.J., Kim, D.E., Isolation of inhibitory RNA aptamers against severe acute respiratory syndrome (SARS) coronavirus NTPase/Helicase. Biochem Biophys Res Commun. 2007 Dec 17].

The patent application WO2007043784 (publ. 2007-04-19) relates to RNA aptamers and uses thereof, more precisely RNA aptamers interfering the interaction of TCF with other proteins by binding specifically to ss-catenin, RNA aptamers binding specifically to HMG domains of TCF-I proteins and uses of the same. The RNA aptamer of the present invention can be effectively used for the development of an anticancer agent since it binds specifically to TCF-I to interrupt the interaction of TCF with ss-catenin involved in tumorigenesis and metastasis and the transcriptional activity of TCF-I in relation to oncogenes.

The patent application US6995249 (publ. 2006-02-07) relates to a novel nucleic acid (aptamer) which binds specifically to the target protein of Ras, more particularly, a novel RNA aptamer which binds specifically to Raf-1; a method for screening an RNA capable of binding specifically to the target protein of Ras which comprises selecting an RNA capable of binding to the target protein of Ras from a pool of RNAs having various base sequences; a method for regulating the signal transduction causing the proliferation or differentiation of cells by using the above-described nucleic acid; and medicinal compositions with the use of the same.

The patent application US2005202423 (publ. 2005-09-15) disclosed a method for identifying compounds (lead structures) which specifically bind to (a) desired RNA target motif and can inhibit or eliminate the function thereof or (b) suppress a compound associated with a desired RNA target motif and can thereby inhibit or eliminate the function thereof. The inventive method is based on the attachment of a ligand (=a compound to be identified) to a RNA target motif which is coupled to a modified ribozyme so that the ribozyme is transformed into an active or inactive conformation resulting in the cleaving of a signal-giving ribozyme substrate. The identified compounds enabling modification of the cellular function of the RNA target motifs enable specific medicaments to be produced. The invention also relates to a polynucleotide comprising a hammerhead ribozyme and an aptamer for a target molecule.; The base pairing model of the polynucleotide, when the target molecule binds to the aptamer, is different from the base pairing model of the polynucleotide when the target molecule does not bind to the aptamer.

The patent application US2005239061 (publ. 2005-10-27) relates to the construction of an allosteric control module in which a catalytic RNA forms a part of or is linked to an effector-binding RNA domain or aptamer. These constructs place the activity of the catalytic RNA under the control of the effector and require the presence of an appropriate effector for activation or inactivation. The present invention provides means to identify useful effector molecules as well as their use to evolve cognate aptamers. The invention involves both the evolution of RNA sequences which bind the effector and a selection process in which the allosteric control modules are identified by their catalytic function in the presence and absence of the effector. The resulting regulatable catalytic RNAs may be used to alter the expression of a target RNA molecule in a controlled fashion.

As has been mentioned earlier, miRNAs play an extraordinarily significant role in regulating gene expression, especially in such processes as cell differentiation and proliferation, maturing, apoptosis, or cancerous transformation. Although the general miRNA functioning principles have already been recognised, we still have no methods that would allow us to actively influence their emergence or functioning. Working out some molecular tools that would allow us e.g. to control the enzymes that take part in miRNA biogenesis may thus be crucial for such areas as medicine (especially treatment of cancer diseases) or biotechnology.

The aim of this invention was to select RNA aptamers that specifically bind human ribonuclease Dicer, which is one of the main enzymes that take part in miRNA biogenesis and the biogenesis of other short regulatory RNAs, and then to find out if pre-selected molecules affect enzyme activity. Other aims of this solution include: (i) using RNA aptamers as ribonuclease Dicer competitive inhibitors; (ii) using RNA aptamers that are allosteric ribonuclease Dicer inhibitors, (iii) identifying RNA aptamers that are selective inhibitors of emergence of selected miRNAs.

This invention fulfils the aims described as above, and solves problems related with the possibility to use RNA aptamers as ribonuclease Dicer competitive inhibitors and as allosteric ribonuclease Dicer inhibitors, as well as selective inhibitors of emergence of the selected miRNAs.

The subject matter of this invention is the in vitro method to inhibit ribonuclease Dicer, wherein ribonuclease Dicer is inhibited by means of RNA aptamer, characterised in that it is selected from the SEQ ID. NO. 1 to 4 and it includes the selection process of RNA aptamers that specifically bind ribonuclease Dicer, checking the influence of preselected aptamers upon enzyme activity wherein flanking sequences of pre-selected RNA aptamers contain from 10 to 30 nucleotides, while the centrally situated random sequence contains from 10 to 30 nucleotides and using pre-selected aptamers to inhibit Dicer activity ribonuclease, whereas each preselected RNA aptamer contains two flanking sequences each and a centrally situated random sequence, and wherein ribonuclease Dicer is inhibited by means of the aptamer that acts upon the competition basis or the RNA aptamer, which is an allosteric inhibitor or the aptamer that acts as a selective or non-selective inhibitor of emergence of the selected miRNAs. Preferably, pre-miRNA cutting efficiency decreases together with increase in RNA aptamer concentration, whereas the scope of the aptamer's molar excess as compared to Dicer is contained within range from 1 to 100. Preferably, mammalian, preferably human ribonuclease Dicer, is inhibited.

The next subject matter of this invention is a ribonuclease Dicer inhibitor, wherein the inhibitor is an RNA aptamer, which has been obtained in the way of *in vitro* selection. and which binds ribonuclease Dicer, and it contains two flanking sequences and a centrally situated random sequence, wherein flanking sequences contain from 10 to 30 nucleotides, while the central random sequence contains from 10 to 30 nucleotides and it is selected from the SEQ ID. NO 1 to 4. Preferably, inhibitor acts upon the competition basis. Preferably, the inhibitor is an allosteric inhibitor. Preferably, inhibitor selectively or non-selectively inhibits emergence of the selected miRNAs.

The next subject matter of this invention is use of RNA aptamers, in which RNA aptamers are selected from the SEQ ID. NO 1 to 4 and are obtained in the way of *in vitro* selection, and contain two flanking sequences each and the centrally situated random sequence in which flanking sequences of preselected RNA aptamers contain from 10 to 30 nucleotides, while the centrally situated random sequence contains from 10 to 30 nucleotides to inhibit ribonuclease Dicer.
Preferably, pre-miRNA cutting efficiency decreases together with increase in aptamer concentration, whereas the scope of the aptamer's molar excess as compared to Dicer is contained within range from 1 to 100. Preferably, ribonuclease Dicer is inhibited by means of the aptamer that acts upon the competition basis. Preferably, ribonuclease Dicer is inhibited by means of the RNA aptamer that is an allosteric inhibitor. Preferably, ribonuclease Dicer is inhibited by means of aptamer that acts as selective or non-selective inhibitor of emergence of the selected miRNAs. Preferably, mammalian, preferably human ribonuclease Dicer, is inhibited.

Drawings enclosed at the end of this study facilitate better understanding of the discussed issues, and illustrate the subject matters of this invention.
Figure **1** presents the ssDNA combinatorial library and starter sequences used in *in vitro* selections. ATD2 and ATD3 starters were used to obtain dsDNA that constituted a template for the *in vitro* transcription reaction. The ATD2 starter additionally contained RNA T7 polymerase promoter. ATD2e and ATD3p starters were used to obtain dsDNA, which was cloned to a plasmid, thus both of these starters contained sequences that were recognised by restrictive enzymes, EcoRI and PstI respectively.
Figure **2** presents the influence of ATD15.52 upon Dicer activity:
   A. Influence of ATD15.52 upon pre-hsa-miR33a cutting.
   B. Influence of ATD 15.52 upon pre-hsa-miR210 cutting.
      In reactions marked with number 1, the aptamer: Dicer molar relation was 1:1, in reactions marked with number 2 it was 10:1, and in reactions marked with number 3 it was 100:1. Additionally, two control reactions were always carried out, i.e.: K+ - standard reaction with no aptamer addition and K- standard reaction with no enzyme. C. Cutting efficiency determined for both of these substrates by Dicer (average from three independent experiments).

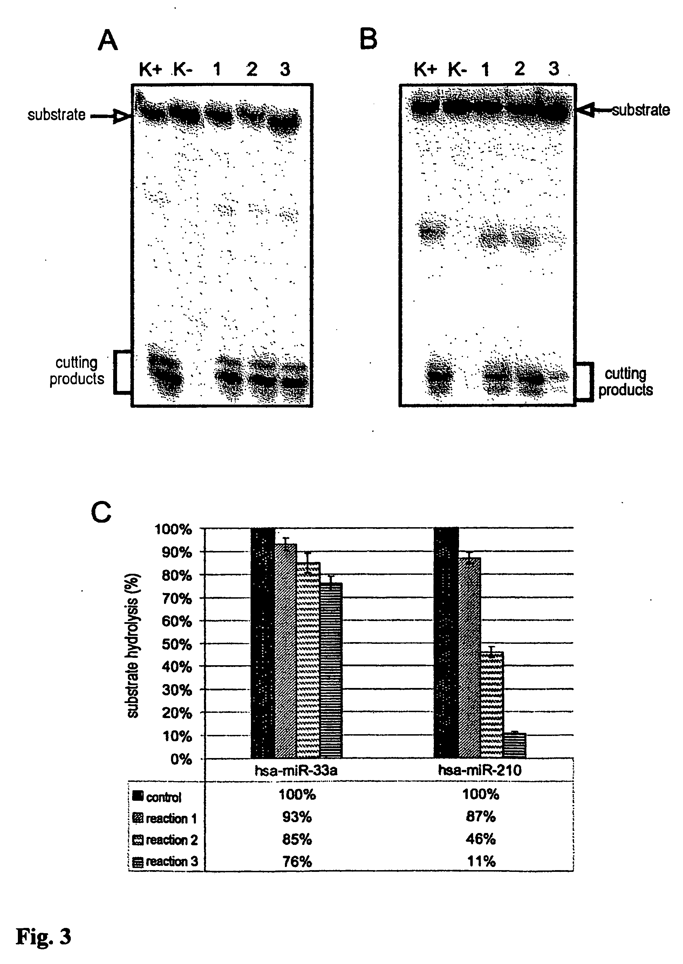

Figure 3 presents the influence of ATD13.6 upon Dicer activity.
   A. Influence of ATD13.6 upon pre-hsa-miR33a cutting.
   B. Influence of ATD 13.6 upon pre-hsa-miR210 cutting.
      In reactions marked with number 1, the aptamer : Dicer molar relation was 1:1, in reactions marked with number 2 it was 10:1, and in reactions marked with number 3 it was 100:1. Additionally, two control reactions were conducted in each case, i.e. - K+ - standard reaction with no aptamer addition and K- standard reaction with no enzyme. C. Cutting efficiency determined for both of these substrates by Dicer (average from three independent experiments).

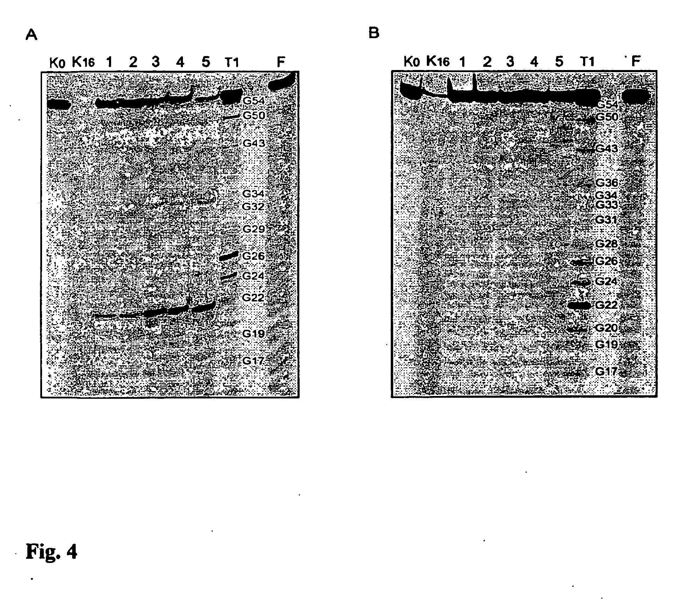

**Figure 4** presents the results of digestion of ATD15.52 (A) and ATD13.6 (B) aptamers using ribonuclease Dicer.
   Lanes, for which reaction mixtures were incubated for 10', 1h, 2h, 5h and 16 h were labelled as 1, 2, 3, 4 and 5, respectively.
   T1 - aptamer cut by ribonuclease T1, F - aptamer that underwent formamide hydrolysis. K0 - control reaction with no enzyme, K16 - control reaction with no enzyme after 16h incubation.
   Presented below are example embodiments of the present invention defined above.

### Example

Ribonuclease Dicer is one of the key enzymes that take part in both miRNA biogenesis and biogenesis of other short regulatory RNAs. It cuts functional miRNA molecules out of double-strand pre-miRNA precursors. Therefore, a question was raised if activity of the enzyme that is responsible for generating short regulatory RNAs, including miRNAs, can be controlled by other RNA molecules. In order to solve this problem, we decided to conduct selection of RNA aptamers that specifically bind human ribonuclease Dicer, and then to check if pre-selected molecules affect this enzyme's activity.

### In vitro selection

### Obtaining a combinatorial library of single-strand RNAs

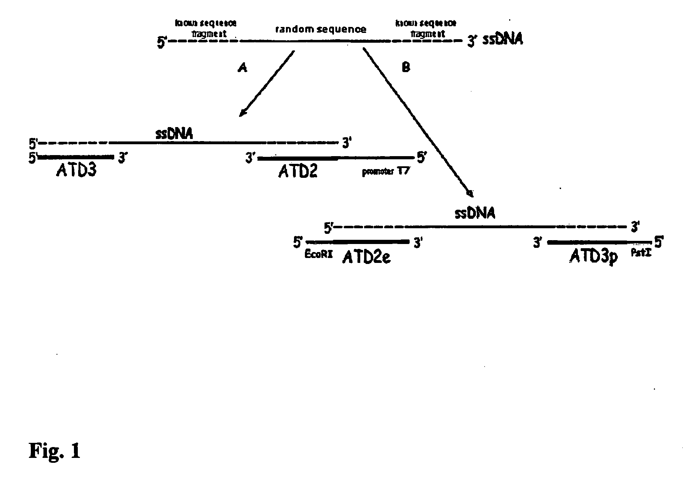

At first, a combinatorial library of single-strand DNAs (ssDNAs) was obtained (as shown in Fig. 1). It was obtained by chemical synthesis. Then, ssDNA library was converted into double-strand DNA (dsDNAs) library by PCR involving ATD2 and ATD3 starters. The PCR reaction was carried out in Biometra® apparatus.

**Table 1. High scale PCR - reaction mixture**

| **Constituent** | **Initial concentration** | **final concentration** |
|---|---|---|
| Template | 0.01 *µ*g/*µ*l | 25 pg/*µ*l |
| 5' starter | 25 pmole/*µ*l | 2.0 pM/*µ*l |
| 3' starter | 25 pmole/*µ*l | 2.0 pM/*µ*l |
| Reaction buffer' | 10x | 1x |
| dNTPs mixture | Cₚ=2.5 mM | Cₖ=0.2 mM |
| MgCl₂ | Cₚ=25 mM | Cₖ=1.5 mM |
| Polymerase DNA Taq | 5U/*µ*l | 5U |
| H₂O | added² to obtain the volume of 400 *µ*l | |
| ¹Reaction buffer: 75 mM Tris-HCl (pH 8.8 at 25°C), 20 mM (NH4)2SO4, 0.01% (v/v) Tween 20 | | |
| ²In order to ensure proper PCR conditions, the reaction mixture was divided into eight parts at least, so that a single test-tube contained maximum 50 *µ*l of the mixture. | | |

**Table 2. High scale PCR - reaction conditions**

| **STAGE** | **TEMPERATURE** | **TIME** | **NUMBER OF REPETITIONS** |
|---|---|---|---|
| Initial denaturation | 93°C | 30 sec. | 1 |
| Denaturation | 93°C | 30 sec. | 30 |
| Hybridisation | 52°C | 30 sec. | |
| Elongation | 72°C | 1 min. | |
| Final elongation | 72°C | 5 min. | 1 |

The dsDNAs obtained served as a template to synthesise the combinatorial library of single-strand RNAs (ssRNAs) by the *in vitro* transcription method. The transcription was carried out in the volume of 50 *µ*l. and the reaction mixture with composition as given in Table 3 was incubated for 4 hours at 37°C. After this time, RNase-free DNase was added into the reaction mixture in order to remove the DNA template.

**Table 3. In vitro transcription - reaction mixture**

| **Constituent** | **initial concentration** | **final concentration** |
|---|---|---|
| Buffer | 5x | 1x |
| NTPs mixture | 25 mM | 2 mM |
| Guanosine | 15 mM | 4 mM |
| Template | 5 pmole/*µ*l | 0.5 pM/*µ*l |
| H₂O | added to obtain the final volume of 50 *µ*l | |

After transcription, the RNA was purified in 12% denaturating polyacrylamide gel according to the following procedure:
- products of the *in vitro* transcription reaction were denaturated by heating them up to 95°C for 2-5 min. and rapidly cooling them down on ice, and then they were separated in denaturating polyacrylamide gel,
- gel fragments that contained products of desirable length were cut out and placed in separate test-tubes,
- 150 µl of elution buffer (10% water solution of 3M sodium acetate pH 5) was added to each of the test-tubes, and they were shaken for 1.5h in ambient temperature (this stage was repeated twice),
- fractions collected were joined together, and RNA was precipitated with 3 vol. of 96% ethanol overnight at the temperature of -20°C,
- it was centrifuged for 20 min. at 14000 rpm,
- the solution was decanted, and the pellet was washed with 500 *µ*l 70% of ethanol,
- it then was centrifuged again for 20 min. at 14000 rpm,
- the solution was decanted, and the pellet was dried and dissolved in 20 µl of H₂O,
- purified RNA concentrations were determined by measuring UV light absorption for λ=260.

**At the next stage, RNA molecules creating library were radioisotopically labelled** according to the following procedure:
- ssRNA was denaturated by heating it up to 95°C for 2 min. and rapidly cooling it down on ice (for 10 min). The reaction was carried out in a mixture which composition is presented in Table 4.

**Table 4. ssRNA radioisotopic labelling - reaction mixture**

| **Constituent** | **initial concentration** | **final concentration** |
|---|---|---|
| kinase buffer¹ | 10x | 1x |
| ATP | [γ 32P] 4000-5000 Ci/mmol | 50 *µ*Ci |
| ssRNA | 100 pmole/*µ*l | 0.3 pM/*µ*l |
| T4 kinases | 10 U/*µ*l | 0.5 U/*µ*l |
| H₂O | added to obtain the final volume of 30 *µ*l | |
| ¹ Kinase buffer: 70 mM Tris-HCl ph 7.6, 10 mM MgCl₂, 5 mM DTT. | | |

- the mixture was incubated for 45 min. at 37°C,
- the reaction mixture was diluted to the volume of 200 *µ*l, and 100 *µ*l of phenol and 100 *µ*l of chloroform was added,
- it was then stired with vortex and centrifuged for 1 min. at 14000 rpm,
- nucleic acid-containing water phase was transferred to a new test-tube, and 200 *µ*l of chloroform was added, stired with vortex and centrifuged for 1 min. at 14000 rpm (twice),
- 10 *µ*l of 3M CH3COONa and 3 vol. of 96% ethanol were added into the water phase that had been obtained as a result of the extraction process,
- the solution was incubated overnight at (-20°C),
- it was centrifuged for 20 min. at 14000 rpm,
- the solution was decanted, and the pellet was washed with 500 *µ*l of 70% ethanol,
- it was centrifuged for 20 min. at 14000 rpm,
- the solution was decanted, and the pellet was dried and dissolved in 20 *µ*l of H₂O,
- using the Beckman apparatus, the volume of radioactive material that was introduced into the input RNA pool was determined.

### Selection of Dicer-binding RNAs

### RNA-Dicer binding

The reaction was carried out in the volume of 100 *µ*l in mixture which composition is presented in Table 5; 10-times molar RNA excess was used as compared to protein.

**Table 5 RNA / protein binding - reaction mixture**

| **Constituent** | **Volume** |
|---|---|
| RNA | 250 pM |
| Dicer | 25 pM |
| Buffer 2x | 50 *µ*l |
| H₂O | Added to obtain the final volume of 100 *µ*l |

- the labelled RNA was incubated for 2 min. at 95°C, then slowly cooled down in ambient temperature,
- reaction mixture which composition is presented in Table 5 was prepared,
- the mixture was incubated for 5 min. at 37°C,
- non-bound RNA was washed out on molecular sieves 30kDa Amicon® Ultra-4 Centrifugal Filter Devices made by Millipore (volume 4 ml) at 4000 rpm at 4°C, for washing 5 ml of the bounding buffer was used,
- RNA-Dicer complex that remained on the sieves was transferred to a test-tube and denaturated by adding 200*µ*l 7 M of urea and 400*µ*l of phenol, the whole mixture was shaken out at 1400 rpm, for 20 min. in ambient temperature,
- then it was centrifuged for 10 min. in ambient temperature at 12000 rpm
- the water phase was transferred to a new test-tube and 0.1 of the volume of 3M CH3COONa and 3 volumes of 96% ethanol was added
- the mixture was incubated overnight at (-20°C),
- it was centrifuged for 20 min. at 14000 rpm,
- the solution was decanted, and the pellet was washed in 500 *µ*l of 70% ethanol,
- it was centrifuged for 20 min. at 14000 rpm,
- the solution was decanted, and the pellet was dried and dissolved in 20 *µ*l of H₂O,
- the amount of radioactive material present in the sample was measured - as a result the percentage of the input RNA pool that bound with Dicer was determined.

In order to conduct the next selection cycle, the ssRNA obtained was converted into single- and then double-strand DNA by the RT-PCR method. To this end, 6 *µ*l of water solution of the AT3 starter (25 pmole/*µ*l) was added to the RNA sample that had been obtained in the former selection cycle (dissolved in 20 *µ*l of water), the whole was incubated for 3 min. at 65°C, and cooled down for 10 min. at 4°C. Then, the reaction mixture which composition is presented in Table 6 was prepared. 35 *µ*l of the mixture was added to the RNA sample that included the AT3 starter. The whole was incubated for 1.5h at 42°C.

**Table 6. Reverse transcription - reaction mixture**

| **CONSTITUENT** | **VOLUME** |
|---|---|
| Tris pH 8.0 (1M) | 4 *µ*l |
| KCl (1M) | 4 *µ*l |
| MgCl₂ (200 mM) | 4 *µ*l |
| DTT (80 mM) | 4 *µ*l |
| dNTP (2.5 mM) | 16 *µ*l |
| M-MuL V-RT (200U/*µ*l) | 0.5 *µ*l |
| H₂O | 7.5 *µ*l |

The reverse transcription reaction product obtained was amplified by PCR using ATD2 and ATD3 starters. The PCR reaction was conducted in the Biometra® apparatus. Reaction products were then analysed by electrophoresis in a 12% polyacrylamide gel in denaturating conditions. Concentration of the DNA obtained was determined by measuring UV light absorption for λ=260 nm. The dsDNA obtained constituted a template to synthesise the narrowed RNA pool (by *in vitro* transcription), which underwent the selection process at the successive cycle.

As soon as the 15^{th} selection cycle was over, the obtained RT-PCR product was not converted to RNA, but was cloned into the plasmid in line with methods being generally used. Then, single clones were sequenced. As a result, 50 sequences were identified that corresponded to RNA molecules, which bound with Dicer.

After a thorough bioinformatic analysis of pre-selected RNA's primary and secondary structures, the following 4 aptamers were selected for further examinations:
***aptamer ATD15.14***
***aptamer ATD15.26***
***aptamer A TD 15.52***
***aptamer A TD 13.6***

### Pre-selected aptamers activity testing

Impact of the obtained aptamers upon Dicer activity was tested *in vitro.* To this aim, some standard reactions of human pre-miRNA digestion by Dicer were conducted. Within a single experiment that was carried out at least three times for each aptamer, 4 reactions were conducted: one control reaction (with no aptamer addition) and three reactions with the addition of various aptamer volumes (Dicer : aptamer molar relation was respectively 1:1, 1:10, and 1:100 in these reactions). The two pre-miRNAs previously characterised, i.e. pre-hsa-miR-33a and pre-hsa-miR-210, were selected as substrates (http://microma.sanger.ac.uk/cgi-bin/sequences).
***pre-hsa-miR-33a***
5'gugcauuguaguugcauugcauguucuggugguacccaugcaauguuuccacagugcauca3'
***pre-hsa-miR-210***
5'gccccugcccaccgcacacugcgcugccccagacccacugugcgugugacagcggcug3'

Reactions were conducted in the volume of 10 µl in a commercial ribonuclease Dicer buffer. The pre-miRNA, which was labelled at the 5' end using radioactive phosphorus, was used in this reaction (the labelling was done according to procedure described above). At first, renaturation of aptamers and labelled substrates was carried out separately (by heating them up to 95°C, and then slowly cooling them down to ambient temperature).

At the next stage, Dicer was pre-incubated with a various amounts of the aptamer for 10 min. at 37°C (Dicer : aptamer molar ratio was changing as follow: 1:1, 1:10, and 1:100). Then, the pre-miRNA was added. The digestion reaction was conducted for 10 min. at 37°C. Each reaction was repeated three times for each aptamer. In case of the ATD15.52 aptamer, an analogous series of reactions was additionally carried out, but without pre-incubation.

**Table 7. Testing aptamer impact upon Dicer activity - reaction mixture composition**

| | **control (+)** | **control (-)** | **1** | **2** | **3** |
|---|---|---|---|---|---|
| **aptamer** | ------ | ------ | 1 *µ*l (1 pmole/ *µ*l) | 1 *µ*l (10 pmole/ *µ*l) | 1 *µ*l (100 pmole/ *µ*l) |
| **Dicer** | 1U | ------ | 1U | 1U | 1U |
| **template** | 5000 cpm | 5000 cpm | 5000 cpm | 5000 cpm | 5000 cpm |
| **buffer 5x** | 2 *µ*l | 2 *µ*l | 2 *µ*l | 2 *µ*l | 2 *µ*l |
| **H₂O** | up to 10 *µ*l | up to 10 *µ*l | to 10 | to 10 *µ*l | to 10 |

| | | | | | |
|---|---|---|---|---|---|
| * Buffer composition: 300 mM NaCl, 50 mM Tris-HCl, 20 mM HEPES, 5mM MgCl₂ (pH 9) | | | | | |

As soon as the reaction was over, the products obtained were denaturated by heating them up to 95°C for 5 minutes and rapidly cooling them down on ice, and then they were analysed by electrophoresis in a 15% denaturating polyacrylamide gel. The electrophoretic separation was carried out in the following conditions:
- pre-electrophoresis - 1200 V, 50 W, 10 mA for 10-15min
- proper electrophoresis -1200 V, 50 W, 40 mA for 4h.

After the electrophoretic separation, the products were analysed using a scanner for radioisotopically labelled materials (Phosphorimager, Typhoon 8600).

### Results

Experiments carried out indicated that the ATD15.14 and ATD 15.26 aptamers inhibited Dicer activity in a 30 and 40% degree respectively, when Dicer : aptamer molar ratio was 1 : 100.

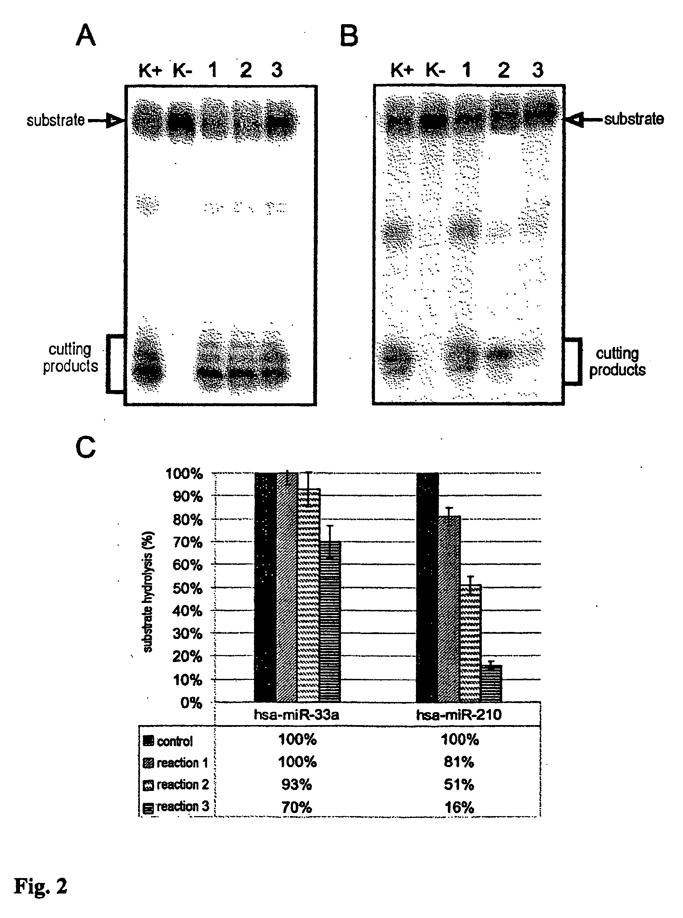

Based upon these experiments, it was found out that ATD 15.52 significantly inhibited the pre-hsa-miR-210 cutting process (when Dicer : aptamer molar ratio was 1 ; 100, only 16% of the substrate was digested), and that it inhibited the pre-hsa-miR33a cutting ( when Dicer : aptamer molar ratio was 1 ; 100, 70% of the substrate was digested) (Figure 2).
It was noticed that together with the increase in aptamer concentration, the pre-hsa-miR-210 cutting efficiency was falling. This inhibitory effect was already observed at aptamer : enzyme molar relation of 1:1, where the number of cutting products tended to fall by 19%. With ten-times molar aptamer excess as compared to the enzyme, about 50% inhibition was observed, and 84% inhibition for 100-times excess. The inhibitor effect was much lower for the pre-miR-33a substrate, reaching 30% inhibition for 100-times excess of ATD15.52.

Similar results were obtained while examining the impact of the ATD13.6 aptamer upon Dicer activity (Figure 3). This aptamer was weak in inhibiting pre-hsa-miR33a digestion (up to 24% maximum), but it was quite strong in pre-hsa-miR210 digestion (up to 89% maximum).

In the next experiments, it was proved that the ATD15.52 aptamer was cut by Dicer, whereas the ATD13.6 was not a substrate for this ribonuclease [compare Fig. 4 and data]. The results obtained allow us to think that the identified aptamers inhibit miRNA emergence according to two different mechanisms. The ATD15.52 aptamer acts as competitor. It binds with Dicer more effectively than pre-hsa-miR210 and is digested, rather than the pre-miRNA. On the other hand, the ATD 13.6 aptamer acts as allosteric inhibitor, i.e. it is not a substrate for Dicer, but it binds with ribonuclease, and inhibits its activity. Interestingly, both of these aptamers inhibited miR210 emergence but not miR33a emergence. This means that it is possible to selectively inhibit the emergence of selected miRNAs by using various aptamers.

## Claims

1. The in vitro method to inhibit ribonuclease Dicer, wherein ribonuclease Dicer is inhibited by means of RNA aptamer, **characterised in that** it is selected from the SEQ ID. NO. 1 to 4 and it includes the conditions of the selection process of RNA aptamers that specifically bind ribonuclease Dicer, checking the influence of preselected aptamers upon enzyme activity, wherein flanking sequences of pre-selected RNA aptamers contain from 10 to 30 nucleotides, while the centrally situated random sequence contains from 10 to 30 nucleotides, and using pre-selected aptamers to inhibit Dicer ribonuclease activity, whereas each preselected RNA aptamer contains two flacking sequences and a centrally situated random sequence and wherein ribonuclease Dicer is inhibited by means of the aptamer that acts upon the competition basis or the RNA aptamer, which is an allosteric inhibitor or the aptamer that acts as a selective or non-selective inhibitor of emergence of the selected miRNAs.

2. The method according to claim 1, wherein pre-miRNA cutting efficiency decreases together with increase in RNA aptamer concentration, whereas the scope of the aptamer's molar excess as compared to Dicer is contained within the range from 1 to 100.

3. The method according to claim 1 or 2, wherein mammalian, preferably human ribonuclease Dicer, is inhibited.

4. The ribonuclease Dicer inhibitor, wherein the inhibitor is an RNA aptamer, which has been obtained in the way of *in vitro* selection, and which binds ribonuclease Dicer, and it contains two flanking sequences and a centrally situated random sequence, wherein flanking sequences contain from 10 to 30 nucleotides, while the central random sequence contains from 10 to 30 nucleotides and it is selected from the SEQ ID No. 1 to 4.

5. Inhibitor according to claim 4, wherein acts upon the competition basis.

6. Inhibitor according to claim 4, wherein the inhibitor is an allosteric inhibitor.

7. Inhibitor according to claim 4, wherein inhibitor selectively or non-selectively inhibits emergence of the selected miRNAs.

8. Inhibitor according to claim 4, wherein it is mammalian, preferably human ribonuclease Dicer inhibitor.

9. Use of RNA aptamers, in which RNA aptamers are selected from the SEQ ID. NO. 1 to 4 and arc obtained in the way of *in vitro* selection and contain two flanking sequences each and the centrally situated random sequence, in which flanking sequences of preselected RNA aptamers contain from 10 to 30 nucleotides, while the centrally situated random sequence contains from 10 to 30 nucleotides, to inhibit ribonuclease Dicer.

10. Use according to claim 9, in which pre-miRNA cutting efficiency decreases together with increase in aptamer concentration, whereas the scope of the aptamer's molar excess as compared to Dicer is contained within range from 1 to 100.

11. Use according to claim 9, in which ribonuclease Dicer is inhibited by means of the aptamer that acts upon the competition basis.

12. Use according to claim 9, in which ribonuclease Dicer is inhibited by means of the RNA aptamer that is an allosteric inhibitor.

13. Use according to claim 9, in which ribonuclease Dicer is inhibited by means of aptamer that acts as selective or non-selective inhibitor of emergence of the selected miRNAs.

14. Use according to claim 9, in which mammalian, preferably human ribonuclease Dicer, is inhibited.

## Patentansprüche

1. *In-vitro*-Verfahren zur Inhibition der Ribonuklease Dicer, wobei Ribonuklease Dicer mit eines RNA-Aptamers inhibiert wird, **dadurch gekennzeichnet, dass** dieses aus SEQ ID NO:1 bis 4 ausgewählt ist und es Bedingungen des Selektionsprozesses der RNA-Aptamere, die Ribonuklease Dicer spezifisch binden, Überprüfen des Einflusses der pre-gewählten Aptamere auf die Enzymaktivität, wobei flankierende Sequenzen der pre-gewählten RNA-Aptamere 10 bis 30 Nukleotide enthalten, während die zentral gelegene zufällige Sequenz 10 bis 30 Nukleotide enthält, und Verwenden der vorausgewählten Aptamere zur Inhibition der Dicer Ribonuklease-Aktivität, wobei jedes vorausgewählte RNA-Aptamer zwei flankierende Sequenzen und eine zentral gelegene zufällige Sequenz enthält und wobei Ribonuklease Dicer mit des Aptamers inhibiert wird, das auf der Grundlage der Konkurrenz wirkt, oder des RNA-Aptamers, das ein allosterischer Inhibitor ist, oder des Aptamers, das als selektiver oder nicht-selektiver Inhibitor der Entstehung von ausgewählten miRNAs wirkt, umfasst.

2. Verfahren nach Anspruch 1, wobei die pre-miRNA-Schneideeffizienz zusammen mit der Zunahme der RNA-Aptamer-Konzentration abnimmt, wobei der Umfang des molaren Überschusses des Aptamers im Vergleich zur Dicer im Bereich von 1 bis 100 liegt.

3. Verfahren nach Anspruch 1 oder 2, wobei Säugetier-Ribonuklease Dicer, bevorzugt menschliche Ribonuklease Dicer, inhibiert wird.

4. Ribonuklease Dicer-Inhibitor, wobei der Inhibitor ein RNA-Aptamer ist, das durch *In*-*vitro*-Selektion erhalten wurde, und das Ribonuklease Dicer bindet, und er zwei flankierende Sequenzen und eine zentral gelegene zufällige Sequenz enthält, wobei flankierende Sequenzen 10 bis 30 Nukleotide enthalten, während die zentral gelegene zufällige Sequenz 10 bis 30 Nukleotide enthält, und er aus SEQ ID NO:1 bis 4 ausgewählt ist.

5. Inhibitor nach Anspruch 4, wobei [er] auf der Grundlage der Konkurrenz wirkt.

6. Inhibitor nach Anspruch 4, wobei der Inhibitor ein allosterischer Inhibitor ist.

7. Inhibitor nach Anspruch 4, wobei der Inhibitor selektiv oder nicht-selektiv die Entstehung von ausgewählten miRNAs inhibiert.

8. Inhibitor nach Anspruch 4, wobei er ein Inhibitor der Säugetier-Ribonuklease Dicer, bevorzugt der menschlichen Ribonuklease Dicer, ist.

9. Verwendung von RNA-Aptameren, wobei RNA-Aptamere aus SEQ ID NO:1 bis 4 ausgewählt sind und durch *In*-*vitro*-Selektion erhalten werden, und jeweils zwei flankierende Sequenzen und die zentral gelegene zufällige Sequenz enthalten, wobei flankierende Sequenzen der vorausgewählten RNA-Aptamere 10 bis 30 Nukleotide enthalten, während die zentral gelegene zufällige Sequenz 10 bis 30 Nukleotide enthält, zur Inhibition der Ribonuklease Dicer.

10. Verwendung nach Anspruch 9, wobei die pre-miRNA-Schneideeffizienz zusammen mit der Zunahme der Aptamer-Konzentration abnimmt, wobei der Umfang des molaren Überschusses des Aptamers im Vergleich zur Dicer im Bereich von 1 bis 100 liegt.

11. Verwendung nach Anspruch 9, wobei Ribonuklease Dicer mithilfe des Aptamers inhibiert wird, das auf der Grundlage der Konkurrenz wirkt.

12. Verwendung nach Anspruch 9, wobei Ribonuklease Dicer mithilfe des RNA-Aptamers inhibiert wird, das ein allosterischer Inhibitor ist.

13. Verwendung nach Anspruch 9, wobei Ribonuklease Dicer mithilfe des Aptamers inhibiert wird, das als selektiver oder nicht-selektiver Inhibitor der Entstehung von ausgewählten miRNAs wirkt.

14. Verwendung nach Anspruch 9, wobei Säugetier-Ribonuklease Dicer, bevorzugt menschliche Ribonuklease Dicer, inhibiert wird.

## Revendications

1. Procédé in vitro pour inhiber la ribonucléase Dicer, dans lequel la ribonucléase Dicer est inhibée au moyen de l'aptamère ARN, **caractérisé en ce qu'**il est sélectionné dans la SEQ ID NO:1 à 4 et il comprend les conditions du processus de sélection des aptamères ARN qui spécialement lient la ribonucléase Dicer, en vérifiant l'influence des aptamères présélectionnés sur l'activité enzymatique, dans lequel les séquences flanquantes des aptamères ARN présélectionnés contiennent de 10 à 30 nucléotides, tandis que la séquence aléatoire située au centre contient de 10 à 30 nucléotides, et en utilisant les aptamères présélectionnés pour inhiber l'activité de la ribonucléase Dicer, tandis que chaque aptamère ARN présélectionné contient deux séquences flanquantes et une séquence aléatoire située au centre et dans lequel la ribonucléase Dicer est inhibée au moyen de l'aptamère qui agit sur la base de concurrence ou l'aptamère ARN qui est un inhibiteur allostérique ou l'aptamère qui agit comme un inhibiteur sélectif ou non sélectif d'émergence des miARNs sélectionnés.

2. Le procédé selon la revendication 1, dans lequel l'efficacité de coupe des pré-miARNs diminue avec l'augmentation de la concentration des aptamères ARN, tandis que la portée de l'excès molaire de l'aptamère par rapport à la Dicer est contenue dans la plage de 1 à 100.

3. Le procédé selon la revendication 1 ou 2, où la ribonucléase Dicer de mammifère, de préférence humaine, est inhibée.

4. L'inhibiteur de la ribonucléase Dicer, où l'inhibiteur est un aptamère ARN qui a été obtenu au moyen de la sélection in vitro, et qui lie la ribonucléase Dicer, et il contient deux séquences flanquantes et une séquence aléatoire située au centre, dans lequel les séquences flanquantes contiennent de 10 à 30 nucléotides, tandis que la séquence aléatoire centrale contient de 10 à 30 nucléotides et il est sélectionné dans la SEQ ID NO:1 à 4.

5. Inhibiteur selon la revendication 4, agissant sur la base de concurrence.

6. Inhibiteur selon la revendication 4, où l'inhibiteur est un inhibiteur allostérique.

7. Inhibiteur selon la revendication 4, où l'inhibiteur inhibe de manière sélective ou non sélective l'émergence des miARNs sélectionnés.

8. Inhibiteur selon la revendication 4, où il est un inhibiteur de la ribonucléase Dicer de mammifère, de préférence humaine.

9. Utilisation des aptamères ARN, dans laquelle les aptamères ARN sont sélectionnés dans la SEQ ID NO:1 à 4 et sont obtenus au moyen de la sélection in vitro, et chacun d'eux contient deux séquences flanquantes et une séquence aléatoire située au centre, dans laquelle les séquences flanquantes des aptamères ARNs présélectionnés contiennent de 10 à 30 nucléotides, tandis que la séquence aléatoire centrale contient de 10 à 30 nucléotides, pour inhiber la ribonucléase Dicer.

10. Utilisation selon la revendication 9, dans laquelle l'efficacité de coupe des pré-miARNs diminue avec l'augmentation de la concentration des aptamères, tandis que la portée de l'excès molaire du aptamère par rapport à la Dicer est contenue dans la plage de 1 à 100.

11. Utilisation selon la revendication 9, dans laquelle la ribonucléase Dicer est inhibée au moyen de l'aptamère qui agit sur la base de concurrence.

12. Utilisation selon la revendication 9, dans laquelle la ribonucléase Dicer est inhibée au moyen de l'aptamère ARN qui est un inhibiteur allostérique.

13. Utilisation selon la revendication 9, dans laquelle la ribonucléase Dicer est inhibée au moyen de l'aptamère qui agit comme un inhibiteur sélectif ou non sélectif d'émergence des miARNs sélectionnés.

14. Utilisation selon la revendication 9, dans laquelle la ribonucléase Dicer de mammifère, de préférence humaine, est inhibée.


### SEQUENCE LISTING

<110> Instytut Chemii Bioorganicznej PAN
   Figlerowicz, Marek
   Kietrys, Anna
   Szopa, Aleksandra
   Tyczewska, Agata
   Twardowski, Tomasz
<120> Method to inhibit ribonuclease Dicer, ribonuclease Dicer
   inhibitor, and use of RNA aptamers as ribonuclease Dicer
   inhibitors
<130> 8560/08/P-R/AT
<140> PCT/PL2009/0000
   <141> 2009-02-06
<150> PL384455
   <151> 2008-02-14
<160> 6
<170> PatentIn version 3.3
<210> 1
   <211> 56
   <212> RNA
   <213> Homo sapiens
<400> 1
   gggagaauca uaaguagccg ggccucccca ucccugcccc auguuaacag uuagcc 56
<210> 2
   <211> 56
   <212> RNA
   <213> Homo sapiens
<400> 2
   gggagaauca uaaguagcac auugaguguu gcccuucccc auguuaacag uuagcc 56
<210> 3
   <211> 56
   <212> RNA
   <213> Homo sapiens
<400> 3
   gggagaauca uaaguagcgc agugagucgu ugugcugccc auguuaacag uuagcc 56
<210> 4
   <211> 56
   <212> RNA
   <213> Homo sapiens
<400> 4
   gggagaauca uaaguagcgg ugugugaguc guggugcccc auguuaacag uuagcc 56
<210> 5
   <211> 61
   <212> RNA
   <213> Homo sapiens
<400> 5
   gugcauugua guugcauugc auguucuggu gguacccaug caauguuucc acagugcauc 60
   a 61
<210> 6
   <211> 58
   <212> RNA
   <213> Homo sapiens
<400> 6
   gccccugccc accgcacacu gcgcugcccc agacccacug ugcgugugac agcggcug 58